# EUROPEAN PATENT APPLICATION

(11) **EP 1 659 182 A1**
(43) Date of publication of application: **24.05.2006**
(21) Application number: 04733216.8
(22) Date of filing: 14.05.2004
(51) Int. Cl.: C12P 19/04

(54) **METHOD FOR PRODUCING OLIGOSACCHARIDE CHITOSAN AND OLIGOSACCHARIDE CHITOSAN**

(30) Priority: 16.05.2003 RU 2003114205
(71) Applicant: Obschestvo s Ogranichennoy Otvetstvennost'yu "Invest-Pharm", Moscow, 127018 (RU)
(72) Inventor: KIRILENKO, Yuriy Karpovich, Moscow, 115522 (RU); FROLOV, Vadim Gennadievich, Nizhny Novgorod, 603054 (RU); NAGAPETIAN, Ruben Armenakovich, Moscow, 125413 (RU); PASTUKHOV, Maksim Olegovich, Nizhny Novgorod, 603148 (RU); CHERKASOVA, Elena Igorevna, Nizhny Novgorod, 603148 (RU); ALEKSEEVA, Maiy Fedorovna, Nizhny Novgorod, 603054 (RU)
(74) Representative: Andrae, Steffen
(86) International application number: PCT/RU2004/000184
(87) International publication number: WO 2004/101804

(57) **Abstract**

The inventions relate to a method of preparing a chitosan oligosaccharide and to the chitosan oligosaccharide product, which may find their use in medical, cosmetic and food-processing industries. It is an object of the invention to provide a method of preparing a chitosan oligosaccharide and the chitosan oligosaccharide product as obtained. A novel chitosan oligosaccharide is **characterized in that** the broad-spectrum ion-bound derivatives thereof make it possible to provide the end product with additional properties and to ensure the stability of said properties under further use. The object is attained by providing an inventive method for preparing a chitosan oligosaccharide consisting in carrying out a fermentative oligomerisation of chitosan in the presence of papain and acetic acid, spay drying the thus enzymatically oligomerised product and, afterwards additionally dissolving the thus obtained product in water at least two times followed by spray drying it again. The thus transformed product is dissolved in water together with substances which contain acid groups, and a salt linkage of the chitosan oligosaccharide is carried out during a solvent process which is carried out until the ion-bound derivatives of the oligosaccharide chitosan are obtained and, afterwards are dried again. The object is also attained by producing a chitosan oligosaccharide containing an ion-bound acetic acid and additionally containing at least one acid groups- containing ion-bound substance. The inventive oligosaccharide chitosan contains 57.0-92.0 % by mass of a oligosaccharide chitosan, 2.3-3.7 % by mass of ion-bound acetic acid, the remaining being an ion-bound substance having acid groups.

## Description

### Field of the invention

The inventions relate to a method of preparing a chitosan oligosaccharide and to the chitosan oligosaccharide product, which may find their use in medical, cosmetic and food-processing industries.

### Background of the invention

A chitosan oligosaccharide is usually obtained by an oligomerisation process. The chitosan, being referred to a typical polysaccharide, is quite easily converted to an oligomer product by using various methods, and more specifically by an enzymatic oligomerisation (see JP Patents 07155193, 11276189, 2001095595).

Known in the art is a method of preparing chitosan oligosaccharides which comprises the step of oligomerisation of chitosan by using enzymes which preferably is papain in the presence of a hydrochloric acid, an acetic acid or an ascorbic acid (see Patent KR 142373).

The disadvantage of the oligosaccharides obtained according to this production process is that the amino groups are wholly linked by the acids used during the enzymatic treatment due to which the range of their use is relatively narrow. Moreover, the disadvantage of this production process is that when preparing, for example ascorbates, there occurs a partial degradation of ascorbic acid under exposure the temperatures due to which the content of it in the resulting product is insufficient. Furthermore, the resulting product is often not stable under storage that results in decreasing its efficiency, if it is used, for example, as a biologically active agent in pharmaceuticals and cosmetic formulations.

### Detailed description of the invention

It is an object of the inventions to provide a method of preparing a chitosan oligosaccharide and the chitosan oligosaccharide product which is characterized by the ion-bound derivatives thereof having broader spectrum of their effects that makes it possible to provide an end product with additional properties and to ensure the stability of said properties during its further use.

According to one object of the present invention there is provided a method of preparing a chitosan oligosaccharide, wherein it comprises the steps of carrying out an enzymatic oligomerisation of chitosan in the presence of papain and an acetic acid after which the enzymatically oligomerised product is firstly subjected to a spray drying, then the spray dried product is dissolved at least two times in water with subsequent the spay drying of it, after which the thus-transformed product is dissolved again in water in combination with the substances having acid groups and during the dissolution process the salt bonding of the chitosan oligosaccharide is carried out and the reaction process is carried out up to the formation of the ion-bound derivatives of the chitosan oligosaccharide, which are then dried again.

According to another object of the invention, the transformed product is dissolved in water followed by adding to the solution at least one acid group-containing substance selected from the groups consisting of a nicotinic acid, a lactic acid, a glutaminic acid, a niflumic acid, a succinic acid, an ascorbic acid, an acexamic acid, glycine hydrochloride , L-carnitine hydrochloride, γ - amino-β-phenylsuccinic acid hydrochloride, adenosine phosphate, cocaborxylase, aspartam, gemfibrozil, an aminocapronic acid hydrochloride, vitamin B₃, methionine.

According to further object to the invention, the dissolution process is carried out by providing a 2-75 % salt linkage of the chitosan oligosaccharide.

According to still another object of the invention, there is provided the chitosan oligosaccharide product, characterized in that it contains an ion-bound acetic acid and additionally comprising at least one acid groups-containing ion-bound substance in the following % by mass:
chitosan oligosaccharide - 57,0 - 92,0,
ion-bound acetic acid 2,3 - 3,7 and
ion-bound substance containing acid groups - the remaining.

According to another object of the invention, there is provided the chitosan oligosaccharide product which comprises at least one acid groups-containing ion-bound substance, selected from the groups consisting of : a nicotinic acid, a lactic acid, a glutaminic acid, a niflumic acid, a succinic acid, an ascorbic acid, an acexamic acid, glycine hydrochloride , L-carnitine hydrochloride, γ - amino-β-phenylsuccinic acid hydrochloride, adenosine phosphate, cocaborxylase, aspartam, gemfibrozil, an aminocapronic acid hydrochloride, vitamin B₃, methionine.

### Description of the embodiments of the invention

### Example 1

The claimed is carried out as follows. The enameled tank is firstly filled with water, then there are added thereto an acetic acid in an amount which gives 1.5 % solution, and chitosan in an amount which provides 5 % content of it in the resulting solution. After the completely dissolving of chitosan in the solution there is added papain in an amount of 0.5 % by mass, based on the weight of chitosan and the reaction is carried out for 5 hours at temperature of 50°C. The resulting solution of the chitosan oligosaccharide is dried in a spray drier PC-18 into which is supplied a hot air at temperature of 160-170°C. The resulting dried product contains 14 % by mass of an ion-bound acetic acid. In so far as the oligosaccharides produced during the enzymatic treatment with acids comprises the amino groups which are wholly linked by acids used at the step of the enzymatic treatment, in particular by an acetic acid used also in the prior art, the content of the acetic acid in the oligosaccharides is rather high at this stage. This makes it impossible of carrying out the step of ionic binding of the thus-obtained oligosaccharides with other substances. As a result, all further steps in the claimed method are directed to decreasing the content of the acetic acid in the thus-obtained oligosaccharides and providing the possibility for further ionic binding them with other substances having acid groups thereby allowing to afford the end product possessing additional biological or other effects, depending on particular substances and the objective put by the manufacturer.

The chitosan oligosaccharide n=2-20 obtained at the step of the enzymatic oligomerisation and dried in a spray dryer is dissolved again in water to afford 5% solution of it and then is it supplied for drying that results in decreasing the content of the acetic acid in it up to 9.2% by mass. The resulting product is dissolved again in water and it is passed through the spray drier under the above-described conditions, resulting in decreasing the content of the acetic up to 5,9 % by mass acid in it, which binds 16.82 % of amino groups of chitosan. Thereafter, an additional binding of amino groups of the chitosan oligosaccharide is carried out with using the substances, containing acid groups and intended to produce the ion-bound oligosaccharides the ion-bound oligosaccharides having this or that activity.For this purpose 100 kg of the thus-obtained chitosan oligosaccharide is dissolved in water to afford a 5 % solution. Thereafter, 56.16 kg of a nicotinic acid is added thereto, which corresponds to a 75 % salt linkage of the chitosan oligosaccharide by the amino
groups and the resulting product is then stirred for a long time until completely uptaking by the oligosaccharide a poorly soluble nicotinic acid. The resulting solution is subjected to a spray drying under the conditions as described above, which affords 151.56 kg of the end product.

The end product had the following content of its ingredients, % by mass:
chitosan oligosaccharide - 61.0.
ion-bound nicotinic acid - 36.45
ion-bound acetic acid - 2.55.

The total content of the ion-bound derivatives were determined by the method of potentiometric titration.

### Example 2.

100 kg of the chitosan oligosaccharide as obtained in a way similar to that as described in Example 1, containing 5.9 % by mass of an acetic acid is dissolved in water to afford a 5 % solution and then 18.72 kg of a nicotinic acid is add thereto, which corresponds to a 25 % salt linkage of the chitosan oligosaccharide by the amino groups and then it is stirred for a long time until completely uptaking by the oligosaccharide a nicotinic acid. The resulting solution similarly as described in Example is passed through a spray drier under the same conditions. As a result, it has been obtained 116.42 kg of the end product.

The end product had the following content of its ingredients, % by mass:
chitosan oligosaccharide - 80.62
ion-bound nicotinic acid - 16.04
ion-bound acetic acid - 3.34.

### Example 3.

100 kg of the chitosan oligosaccharide as obtained under the same conditions as described in Example 1, containing 5.9 % by mass of an acetic acid, is dissolved in water to afford 10% concentration of the solution and then there added to the solution 13.7 kg of an lactic acid, which corresponds to a 25 % salt linkage of the chitosan oligosaccharide by the amino groups. The solution as obtained similarly as described in Example 1 and under the same conditions is passed through a spray drier under the same conditions. As a result, it has been obtained 111.4 kg of the end product.

The end product had the following content of its ingredients, % by mass:
chitosan oligosaccharide - 84.24
ion-bound lactic acid - 12.26
ion-bound acetic acid - 3.5.

### Example 4.

100 kg of the chitosan oligosaccharide as obtained under the same conditions as described in Example 1, containing 5.9 % by mass of an acetic acid, is dissolved in water until it has been reached 10 % concentration of the solution and, thereafter there is added 27.15 kg of glycine hydrochloride which corresponds to a 40 % salt linkage of the chitosan oligosaccharide by the amino groups. The resulting solution is then passed through a spray drier under the same conditions as described in Example 1. As a result, it has been obtained 124.85 kg of the end product.

The end product had the following content of its ingredients, % by mass:
chitosan oligosaccharide - 75.2
ion-bound glycine hydrochloride -21.7
ion-bound acetic acid - 3.1.

### Example 5.

100 kg of the chitosan oligosaccharide as obtained in a way similar to that as described in Example 1, containing 5.9 % by mass of an acetic acid is dissolved in water until it has been reached 10 % concentration of the solution and to the solution there are added 12.03 kg of L-carnitine hydrochloride (3-carboxy-2-hydroxy - N, N, N-trimethyl-1-propaminium hydroxide), which corresponds to a 10 % salt linkage of the chitosan oligosaccharide by the amino groups. The resulting solution is than passed through a spray in a way similar to that as described in Example 1. As a result, it has been obtained 109.73 kg of the end product.

The end product had the following content of its ingredients, % by mass:
chitosan oligosaccharide - 85.52
ion-bound L-carnitine hydrochloride -10.93
ion-bound acetic acid - 3.55

### Example 6.

100 kg of the chitosan oligosaccharide as obtained under the same conditions as described in Example 1, containing 5.9 % by mass of an acetic acid, is dissolved in water until it has been reached 5 % concentration of the solution and, thereafter to the solution there are added 8. 95 kg of glutaminic acid, which corresponds to a 10 % salt linkage of the chitosan oligosaccharide by the amino groups and thereafter the resulting mixture is stirred for a long time until completely uptaking by the oligosaccharide a poorly soluble glutaminic acid. The resulting solution is then passed through a spray drier under the same conditions as described in Example 1. As a result, it has been obtained 106.55 kg of the end product.

The end product had the following content of its ingredients, % by mass:
chitosan oligosaccharide - 87.99
ion-bound glutaminic acid -8.37
ion-bound acetic acid - 3.64

### Example 7.

100 kg of the chitosan oligosaccharide as obtained under the same conditions as described in Example 1, containing 5.9 % by mass of an acetic acid, is dissolved in water until it has been reached 10 % concentration of the solution and, thereafter to the solution there are added 17.9 kg of aspartam (N-L-alpha-aspartam-L-phenylalanine-1-methyl ether, which corresponds to a 10 % salt linkage of the chitosan oligosaccharide by the amino groups. The resulting solution is then passed through a spray drier under the same conditions as described in Example 1. As a result, it has been obtained 115.6 kg of the end product.

The end product had the following content of its ingredients, % by mass:
chitosan oligosaccharide - 81.19
ion-bound aspartam-15.45
ion-bound acetic acid - 3.36

### Example 8.

100 kg of the chitosan oligosaccharide as obtained under the same conditions as described in Example 1, containing 5.9 % by mass of an acetic acid, is dissolved in water until it has been reached 10 % concentration of the solution and, thereafter to the solution there are added 65.24 kg of phenylbut (γ- amino-β-phenylbutyric acid hydrochloride), which corresponds to 50 % salt linkage of the chitosan oligosaccharide by the amino groups. The resulting solution is then passed through a spray drier under the same conditions as described in Example 1. As a result, it has been obtained 163.1 kg of the end product.

The end product had the following content of its ingredients, % by mass:
chitosan oligosaccharide - 57.65
ion-bound phenylbut - 39.96
ion-bound acetic acid - 2.39 %

### Example 9

100 kg of the chitosan oligosaccharide as obtained under the same conditions as described in Example 1, containing 5.9 % by mass of an acetic acid, is dissolved in water until it has been reached 5 % concentration of the solution and, thereafter to the solution there are added 21.1 kg adenosine phosphate (5-adenylic acid), which corresponds to 10 % salt linkage of the chitosan oligosaccharide by the amino groups. The resulting mixture is then is stirred for a long time until completely uptaking by the oligosaccharide a poorly soluble adenosine phosphate. The resulting solution is then passed through a spray drier under the same conditions as described in Example 1. As a result, it has been obtained 118. 8 kg of the end product.

The end product had the following content of its ingredients, % by mass:
chitosan oligosaccharide - 7.0 %
ion-bound adenosine phosphate - 17.72
ion-bound acetic acid - 3.28

### Example 10

10 kg of the chitosan oligosaccharide as obtained in a way similar to that as described in Example 1, containing 5.9 % by mass of an acetic acid, is dissolved in water until it has been reached 10 % concentration of the solution and, thereafter to the solution there are added 0.52 kg of cocarboxylase (3-[(4-amino-2-methyl-5-pyrimidinyl) - methyl] - 4 - methyl-5 (4, 6,6 - trihydroxy - 3, 5 - dioxa-4,6-diphosphahex-1-yl)tluazolium chloride P, P-dioxide) which corresponds 2 % salt linkage of the chitosan oligosaccharide by the amino groups. The resulting solution is then subjected to the liophilization at the freeze drier YCC-25 at a temperature of about 0°C and a residual pressure of 20-25 Poises. As a result, it has been obtained 10.29 kg of the end product.

The end product had the following content of its ingredients, % by mass:
chitosan oligosaccharide - 91.18
ion-bound cocarboxylase - 5.04
ion-bound acetic acid - 3.78

### Example 11

100 kg of the chitosan oligosaccharide as obtained under the same conditions as described in Example 1, containing 5.9 % by mass of an acetic acid, is dissolved in water until it has been reached 10 % concentration of the solution and, thereafter to the resulting solution there are added 15,21 kg of gemfibrozil (2,2-dimethyl-5-(2,5-dimethyl-phenoxy) penthanoic acid), which corresponds to 10 % salt linkage of the chitosan oligosaccharide by the amino groups. Then, the resulting mixture is stirred for a long time until completely uptaking by the oligosaccharide a poorly soluble gemfibrozil. The resulting solution is then passed through a spray drier under the same conditions as described in Example 1. As a result, it has been obtained 112.91 kg of the end product.

The end product had the following content of its ingredients, % by mass:
chitosan oligosaccharide - 83.13
ion-bound gemfibrozil - 13.43
ion-bound acetic acid - 3.44.

### Example 12

100 kg of the chitosan oligosaccharide as obtained in a way similar to that as described in Example 1, containing 5.9 % by mass of an acetic acid, is dissolved in water until it has been reached 10 % concentration of the solution and, thereafter to the solution there are added 20.4 kg of aminocapronic acid hydrochloride that corresponds to a 20 % salt linkage of the chitosan oligosaccharide by the amino groups. The resulting solution is then passed through a spray drier under the same conditions as described in Example 1. As a result, it has been obtained 118.8 kg of the end product.

The end product had the following content of its ingredients, % by mass:
chitosan oligosaccharide - 79.48
ion-bound aminocapronic acid hydrochloride - 17.23
ion-bound acetic acid - 3.29.

### Example 13

100 kg of the chitosan oligosaccharide as obtained in a way similar to that as described in Example 1, containing 5.9 % by mass of an acetic acid, is dissolved in water until it has been reached 5 % concentration of the solution and, thereafter to the solution there are added 15.33 kg of niflumic acids (2-[[3-(trifluoromethyl) phenyl] amino] - 3-pyridinecapronic acid), which corresponds to a 10 % salt linkage of the chitosan oligosaccharide by the amino groups. The resulting solution is then passed through a spray drier under the same conditions as described in Example 1. As a result, it has been obtained 113.01 kg of the end product.

The end product had the following content of its ingredients, % by mass:
chitosan oligosaccharide - 83.03
ion-bound niflumic acid - 13.53
ion-bound acetic acid - 3.44

### Example 14

100 kg of the chitosan oligosaccharide as obtained in a way similar to that as described in Example 1, containing 5.9 % by mass of an acetic acid, is dissolved in water until it has been reached 10 % concentration of the solution and, thereafter, to the solution there are added 53,56 kg of an ascorbic acid (γ - lacton 2,3-dehydro-L-gulonic acid), which corresponds to a 50 % salt linkage of the chitosan oligosaccharide by the amino groups. The resulting solution is then passed through a spray drier under the same conditions as described in Example 1. As a result, it has been obtained 151.26 kg of the end product.

The end product had the following content of its ingredients, % by mass:
chitosan oligosaccharide - 62.09
ion-bound ascorbic acid - 35.34
ion-bound acetic acid - 2.57

### Example 15

100 kg of the chitosan oligosaccharide as obtained in a way similar to that as described in Example 1, containing 5.9 % by mass of an acetic acid, is dissolved in water until it has been reached 10 % concentration of the solution and, thereafter to the solution there are added 35.92 kg of an succinic acid that corresponds to a 50 % salt linkage of the chitosan oligosaccharide by the amino groups. The resulting solution is then passed through a spray drier under the same conditions as described in Example 1. As a result, it has been obtained 133.67 kg of the end product.

The end product had the following content of its ingredients, % by mass:
chitosan oligosaccharide - 70.27
ion-bound succinic acid- 26.82
ion-bound acetic acid - 2.91.

### Example 16

10 kg of the chitosan oligosaccharide as obtained as obtained in a way similar to that as described in Example 1, containing 5.9 % by mass of an acetic acid, is dissolved in water until it has been reached 10 % concentration of the solution and, thereafter to the solution there are added 1,33 kg of vitamin B₃ (D-(+) - 3-(2,4 -dihydroxy-3,3-dimethyl -butyrylamino) propionic acid),which corresponds to a 10 % salt linkage of the chitosan oligosaccharide by the amino groups. The resulting solution is then subjected to the liophilization at the freeze drier at a temperature of about 0°C and a residual pressure of 20-25 Poises. As a result, it has been obtained 11.1 kg of the end product.

The end product had the following content of its ingredients, % by mass:
chitosan oligosaccharide - 84.55
ion-bound vitamin B₃ - 11.95
ion-bound acetic acid - 3.5.

### Example 17

100 kg of the chitosan oligosaccharide as obtained in a way similar to that as described in Example 1, containing 5.9 % by mass of an acetic acid, is dissolved in water until it has been reached 10 % concentration of the solution and, thereafter to the solution there are added 18.5 kg methione, which corresponds to a 20 % salt linkage of amino groups of the chitosan oligosaccharide by the amino groups. The resulting solution is then passed through a spray drier under the same conditions as described in Example 1. As a result, it has been obtained 115.85 kg of the end product.

The end product had the following content of its ingredients, % by mass:
chitosan oligosaccharide - 81.02
ion-bound methionine - 15.63
ion-bound acetic acid - 3.35.

### Example 18

100 kg of the chitosan oligosaccharide as obtained in a way similar to that as described in Example 1, containing 5.9 % by mass of an acetic acid, is dissolved in water until it has been reached 10 % concentration of the solution and, thereafter, to the solution there are added 21.84 kg of acexamic acid, which corresponds to a 20 % salt linkage of the chitosan oligosaccharide by the amino groups. The resulting solution is then passed through a spray drier under the same conditions as described in Example 1. As a result, it has been obtained 119.54 kg of the end product.

The end product had the following content of its ingredients, % by mass:
chitosan oligosaccharide - 79.72
ion-bound acexamic acid - 17.13
ion-bound acetic acid - 3.15.

The obtained chitosan oligosaccharides were tested in the Nizhniy Novgorod State Medical Academy. The efficiency of the pharmaceutical compositions as produced was confirmed by the results of their testing in comparison with the oligosaccharides, containing no ion-bound derivatives of the substances having acid groups. The results of testing showed a considerable positive effect by using the chitosan oligosaccharides comprising the ion-bound ascorbic and uccinic acids in the treatment of patients suffered from intoxication associated by the action of alcohol, in the therapy of dislipoproteidemia, an atherosclerosis and also in the treatment of a tuberculosis, an enzymatic system of a liver and a vertebral dorsalgia.

The chitosan oligosaccharides, cocarboxylase, adenosine phosphate, vitamin B₃, methionine, gemfibrozil, a niflumic acid, an acexamic acid, phenbut have demonstrated higher stability of their biologically active properties of these compounds under storage due to chemical linkage with oligomeric carrier.

### Industrial applicability

The advantages of the claimed inventions are that they make it possible to obtain the ion-bound derivatives of a chitosan oligosaccharide which, if used the substances of the closest prior arts cannot be produced such as, for example, hydrochloride carnitine, aspartam, vitamin B₃, glycine hydrochloride. Moreover, the conditions of the claimed inventions provide the stability of those substances, such as acetylsalicylic acid, vitamin B₃, L-carnitine, aspartam, antibiotics, cocarborxylase, methionine and the like, which under the conditions of the closest prior art are usually underwent the degradation under affecting the enzymes. Thus the claimed method makes it possible to obtain the oligosaccharides with the ion-bound derivatives due to which the final oligosaccharides possess a several number of effects, such as a biological activity (an antineoplastic effect, hepatoprotective effects), and also the characteristics of ion-bound compounds thereof, with attaining in several cases the specific synergism, the stabilization and the stability of these characteristics, and the demonstration of the whole complex of properties, such, as an antineoplastic effect, hepatoprotective and antidiabetic properties.

The described advantages provides a broader spectrum of the claimed method action and makes it possible to obtain a great variety of a biologically active compounds having a wide spectrum of their action which may find their use in preparing the pharmaceuticals, vitamins, amino acids, including antibiotics and also the substances suitable for food and cosmetic purposes.

## Claims

1. A method of preparing a chitosan oligosaccharide by carrying out the step of an enzymatic oligomerisation of chitosan in the presence papain and an acetic with the subsequent drying of the resulting product, **characterized in that** after the enzymatic oligomerisation the resulting product is firstly subjected to a spray drying and after the spray drying the resulting product is then dissolved at least two times in water followed by the spray drying after which the thus-transformed product is dissolved again in water in combination with the substances having acid groups and during the dissolution process the salt linkage of the chitosan oligosaccharide is carried out, and the reaction process is carried out up to the formation the ion-bound derivatives of the chitosan oligosaccharide, and the resulting derivatives are then subjected to drying again.

2. A method according to claim 1, **characterized in that** after which the thus- transformed product is dissolved in water by adding to the solution at least one substance having acid groups, selected from the groups consisting of : a nicotinic acid, a lactic acid, a glutaminic acid, a niflumic acid, a succinic acid, an ascorbic acid, an acexamic acid, glycine hydrochloride , L-carnitine hydrochloride, γ- amino-β-phenylsuccinic acid hydrochloride, adenosine phosphate, cocaborxylase, aspartam, gemfibrozil, aminocapronic acid hydrochloride, vitamin B₃, methionine.

3. A method according to claim 1, **characterized in that** the dissolution process is carried out by providing a 2-75 % salt linkage of the chitosan oligosaccharide.

4. The chitosan oligosaccharide **characterized by** the presence of an ion-bound acetic acid, wherein it further contains at least one ion-bound substance having acid groups in the following % by mass;
chitosan oligosaccharide - 57.0 - 92.0
ion-bound acetic acid - 2.3 - 3. 7 and
acid groups-containing ion-bound substance - the remaining.

5. The chitosan oligosaccharide, **characterized in that** it contains at least one acid group-containing ion-bound substance selected from the groups consisting of a nicotinic acid, a lactic acid, a glutaminic acid, a niflumic acid, a succinic acid, an ascorbic acid, an acexamic acid, glycine hydrochloride, L-carnitine hydrochloride, γ-amino-β-phenylsuccinic acid hydrochloride, adenosine phosphate, cocarboxylase, aspartam, gemfibrozil, aminocapronic acid hydrochloride, vitamin B₃, methionine.
